Europäisches Patentamt

European Patent Office

Office européen des brevets

(19)

(11) Veröffentlichungsnummer: **0 272 562 B1**

(12) # EUROPÄISCHE PATENTSCHRIFT

(45) Veröffentlichungstag der Patentschrift: **09.03.94**

(51) Int. Cl.5: **A61L 15/16**, **C09J 4/00**

(21) Anmeldenummer: **87118420.6**

(22) Anmeldetag: **11.12.87**

(54) **Basische, wirkstoffdurchlässige haftklebende Polymermasse, Verfahren zu ihrer Herstellung und ihre Verwendung.**

(30) Priorität: **22.12.86 DE 3643987**

(43) Veröffentlichungstag der Anmeldung:
**29.06.88 Patentblatt 88/26**

(45) Bekanntmachung des Hinweises auf die
Patenterteilung:
**09.03.94 Patentblatt 94/10**

(84) Benannte Vertragsstaaten:
**AT BE CH DE ES FR GB GR IT LI LU NL SE**

(56) Entgegenhaltungen:
**EP-A- 0 155 229**
**EP-A- 0 156 080**
**EP-A- 0 225 005**
**EP-A- 0 242 827**
**WO-A-86/00814**

(73) Patentinhaber: **LTS Lohmann Therapie-Systeme GmbH & Co. KG**
**Postfach 23 43**
**D-56513 Neuwied(DE)**

Patentinhaber: **SANDOZ AG**
**Lichtstrasse 35**
**CH-4002 Basel(CH)**

(72) Erfinder: **Müller, Walter, Dr.**
**Engerser Strasse 56**
**D-5450 Neuwied 1(DE)**
Erfinder: **Czech, Zbigniew, Dr.**
**Rostocker Strasse 10**
**D-5400 Koblenz(DE)**
Erfinder: **Simon, Günter**
**Tulpenweg 1**
**D-5533 Hillesheim(DE)**
Erfinder: **Reinhardt, Joerg, Dr.**
**Schauinslandstrasse 5**
**D-7801 Ehrenkirchen(DE)**

(74) Vertreter: **Neidl-Stippler, Cornelia, Dr.**
**Rauchstrasse 2**
**D-81679 München (DE)**

EP 0 272 562 B1

**Beschreibung**

Die Erfindung betrifft die Kombination einer basischen wirkstoffdurchlässigen haftklebenden Polymermasse mit basischen Wirkstoffen, ein Verfahren zu ihrer Herstellung sowie ihre Verwendung.

Hautverträgliche haftklebende Polymermassen auf Acrylatbasis zur Befestigung von einfachen Pflastern auf der Haut unter Verwendung von bis zu 25 Gew.% Dimethylaminoethylmethacrylat sowie anderer basischer Amin-Monomere in den Ausgangsmaterialien sind bspw. aus der GB-PS 1 166 682 und der US-A-3 790 533 bekannt. Dabei dient in der GB-PS 1 166 682 das Diethylaminoethylmethacrylat zur Viskositätseinstellung der Latex, während in der US-A-3 790 533 das Diethylaminoethylmethacrylat in zu in der Polymerausgangsmischung vorhandener freier Säure stöchiometrischer Menge zugesetzt wird, um diese zu neutralisieren.

Basische Polymere werden auch in transdermalen therapeutischen Systemen eingesetzt, die es erlauben, systemisch oder topisch wirkende Arzneistoffe transdermal zu verabreichen.

Derartige transdermale Systeme bestehen im wesentlichen aus einer für den Wirkstoff und ggf. Hilfsstoffe undurchlässigen Trägerfolie, einer haftklebenden Schicht und häufig auch einem dazwischenliegenden Wirkstoffreservoir. Die haftklebende Schicht muß nicht notwendigerweise das Wirkstoffreservoir vollständig bedecken, und ist in speziellen Fällen lediglich als ein Kleberand ausgeführt. Zwischen dem Wirkstoffreservoir und der haftklebenden Schicht bzw. der Haut kann sich noch eine die Wirkstoffabgabe steuernde Membran befinden. Derartige therapeutische Systeme sind bspw. in den US-A-en 3 734 097; 3 598 112; 3 249 109; 3 797 494; der EP-PS 0 033 615 und den DE-PSen 21 35 533 und 28 22 317 beschrieben.

Aus der EP-A-155 229 sind basische wirkstoffdurchlässige und zugleich haftklebende Polymermassen bekannt, bei denen das betreffende Polymer unter Verwendung bestimmter basischer Acrylmonomere hergestellt ist.

Es handelt sich dort insbesondere um spezielle hydrophile quellbare basische Polymere, die gemeinsam mit basischen Wirkstoffen, nämlich Bopindolol, Tizanidin, Clemastin, Ketotifen oder Methysergid verwendet werden.

Die EP-A-155 229 lehrt punktuell eine basische hydrophile quellfähige Matrix gemeinsam mit basischen Wirkstoffsalzen und gibt lediglich Informationen über die Quellfähigkeit der verwendeten Polymere - weitergehende allgemeine Erkenntnisse über die Kombination basischer Polymere mit basischen Wirkstoffen sind nicht enthalten.

Die EP-A-156 089 beschreibt ein System, das möglichst viel Wirkstoff im System selbst behalten kann und strebt eine Salzbildung innerhalb der Matrix an.

Die WO 86/00814 beschreibt allgemein die Herstellung vernetzter haftklebender Acrylverbindungen mit Wirkstoffen. Dort sind weder basische Acrylverbindungen angegeben noch eine Anregung, wie diese mit basischen Wirkstoffen zu verwenden sind.

Die EP-A-242 827, die nicht vorveröffentlicht ist, schlägt lediglich punktuell vor, Bopindolol aus in Wasser quellbaren basischen Polyacrylaten freizusetzen, wie dies auch aus der EP-A-155 229 bekannt ist, offenbart also punktuell die Kombination von Bopindolol mit in Wasser quellbaren basischen Polyacrylaten.

Die EP-A-225 005, die ebenfalls erst nach dem Prioritätstag der Anmeldung veröffentlich wurde, betrifft die Kombination von sauren Matrices mit den Salzen saurer Wirkstoffe, um aus dem Salz eines sauren Wirkstoffes die besser durch die Haut penetrierende freie Wirkstoffsäure freizusetzen - also ein saures System.

Klebende Materialien, die zur Befestigung von wirkstoffhaltigen Reservoirs auf einem Untergrund, an den sie den Wirkstoff in einer vorherbestimmten Geschwindigkeit abgeben, dienen, sind bekannt.

Im einfachsten Fall ist die haftklebende Schicht mit dem Wirkstoffreservoir identisch. Die haftklebende Schicht dient dazu, das transdermale System für die Dauer der Applikation auf der Haut zu verankern. Das klebende Wirkstoffreservoir bzw. die Klebeschicht eines derartigen Systems kann vor Anwendung durch eine abziehbare Schutzfolie geschützt sein. Die Wanderung des Wirkstoffes aus dem transdermalen System in und durch die Haut erfolgt durch passive Diffusion. Daraus ergibt sich unmittelbar, daß eine gute Diffusionsgeschwindigkeit des Wirkstoffes im transdermalen System für dessen Funkton von größter Wichtigkeit ist.

Entscheidend für die Funktion dieser transdermalen Systme ist, daß der Wirkstoff die lipophile Barriere der menschlichen Hornhaut passieren kann. Um dies zu erreichen, muß ggf. ein als Penetrationsbeschleuniger bezeichneter Hilfsstoff eingesetzt werden.

In der EP-A-0155229 ist vorgeschlagen worden, hydrophile quellbare Polymere aufweisende basische Wirkstoffreservoirs mit Wirkstoffen einzusetzen, um durch die Wasseraufnahme des Polymeren aus der Haut den Wirkstofftransport zu beschleu-nigen. Die Lehre der EP-A-0155229, Polymere hydrophil und

2

quellbar zu machen und dadurch die Abgabe der Wirkstoffe zu verbessern, führte jedoch immer noch nicht zu zufriedenstel-lenden Resultaten für die Wirkstofffreisetzung.

Viele medizinische Wirkstoffe enthalten ein oder mehrere basische Stickstoffatome im Molekül und können deshalb entweder als freie Base oder als Salz der Wirkstoffbase mit einer für diesen Zweck geeigneten Säure in pharmazeutischer Zubereitung eingesetzt werden. Salze haben den Vorteil besserer Löslichkeit in wäßrigen Lösemitteln und in vielen Fällen auch den der besseren Stabilität, so daß die Salze häufig der Endpunkt vieler Arzneistoffsynthesen (wichtig für die orale Verabreichung) sind.

Für die transdermale Verabreichung von solchen systemisch wirkenden Arzneistoffen ist jedoch in der Mehrzahl der Fälle die für die Lagerung oft ungeeignetere Base dem Salz überlegen, da sie aufgrund ihrer höheren Lipophilie leichter die lipophile Barriere der menschlichen Hornhaut durchdringen kann.

Es ist demnach Aufgabe der vorliegenden Erfindung, eine, die Abgabe von Wirkstoffen aus Matrices zu verbessern. Die Aufgabe wird erfindungsgemäß durch die Kombination einer basischen wirkstoffdurchlässigen haftklebenden Polymermasse mit basischen Wirkstoffen, wobei die haftklebende Polymermasse ein unter Verwendung von basischen Monomeren hergestelltes Homo- oder Copolymeres, bevorzugt auf Actylatbasis, ausge-schlossen die Kombination wirkstoffdurchlässiger hydrophiler quellbarer basischer haftklebender Polymere mit Bopindolol, Tizanidin, Clemastin, Ketotifen oder Methysergid ist.

Dadurch, daß ein durch Einpolymerisation basische Eigenschaften aufweisendes klebendes Polymeres eingesetzt wird, kann bspw. in zumindest teilweise aus der erfindungsgemäßen Zusammensetzung aufgebauten klebenden Wirkstoffreservoirs die Wirkstoffbase aus Wirkstoffsalzen freigesetzt werden und als lipophile Base durch die Haut diffundieren.

Eine bevorzugte Ausführungsform einer Kombination einer basischen Polymermasse mit basischem Wirkstoff ist erhältlich durch an sich bekannte Polymerisation von:

a) zwischen etwa 30 bis 70 Gew.%, bevorzugt 40 bis 60 Gew.%, eines oder mehrerer Alkylester der Acryl- und/oder Methacrylsäure mit 4 - 12 C-Atomen im Alkylrest;

b) 5 bis 85 Gew.%, bevorzugt 15 bis 50 Gew.%, eines oder mehrerer Aminoester der Acryl- und/oder Methacrylsäure oder anderer in ein Polyacrylat einpolymerisierbarer Olefine mit basischen Gruppen;

c) 0 bis 50 Gew.%, bevorzugt 5 bis 20 Gew.%, eines oder mehrerer Alkylester der Acryl- und/oder Methacrylsäure mit 1 bis 3 C-Atomen im Alkylrest;

d) 0 bis 15 Gew.%, bevorzugt 0,5 bis 12,5, besonders bevorzugt 1,0 bis 9, und ganz besonders bevorzugt 5 bis 8, Gew.% eines oder mehrerer einpolymerisierbarer Monomere mit für Nachvernetzung geeigneten, reaktionsfähigen Gruppen;

e) 0 bis 30 Gew.%, bevorzugt 2 bis 15 Gew.%, von anderen einpolymerisierbaren Vinylverbindungen; und

f) 0 bis 5 Gew.-%, bevorzugt 0,3 Gew.-%, von einpolymerisierbaren Divinylverbindungen.

Vorteilhafte Weiterbildungen ergeben sich aus den Unteransprüchen 3 bis 8, auf die hiermit zur Vermeidung von Wiederholungen Bezug genommen wird.

Ferner bezieht sich die Erfindung auf ein Verfahren zur Herstellung einer Zusammensetzung nach den Ansprüchen 1 bis 8, wie in Anspruch 9 beansprucht.

Ferner bezieht sich die Erfindung auf die Verwendung der erfindungsgemäßen Kombination gemäß den Ansprüchen 10 bis 13.

Dabei kann der therapeutisch wirksame Teil des Wirkstoffsalzes ein oder mehrere basische Stickstoffatome enthalten. Eine bevorzugte Verwendung ist der Einsatz der erfindungsgemäßen Polymermasse mit Bopindolol und seinen Derivaten.

Basizität und Menge der einpolymerisierten basischen Monomere legen über das sich einstellende chemische Gleichgewicht fest, welcher Anteil Wirkstoff jeweils im System als freie Base vorliegt.

Dieses Gleichgewicht stellt sich, wenn während der Anwendung freie Wirkstoffbase aus dem System diffundiert, immer wieder neu ein, so daß, vorausgesetzt die dafür notwendige Mindestmenge an basischen Hilfsgruppen wird nicht unterschritten, letztendlich der gesamte als Salz eingesetzte Wirkstoff in die freie Base überführt wird.

Aufgrund des unterschiedlichen Permeationsvermögens von Wirkstoffsalz und Wirkstoffbase durch die menschliche Haut ergibt sich damit die neue Möglichkeit, die in-vivo-Freisetzung des Wirkstoffs aus dem transdermalen System durch die Beeinflussung des chemischen Gleichgewichts über Variation der Basizität und Menge der einpolymerisierten Monomeren mit funktionellen basischen Gruppen zu steuern.

Grundsätzlich lassen sich alle für die medizinische Verwendung geeigneten klebenden Polymer-Zusammensetzungen in der oben beschriebenen Art und Weise modifizieren, wobei Monomere mit basischen funktionellen Gruppen einpolymerisiert werden.

Dabei kann es notwendig sein, in die fertige klebende Masse Substanzen einzuarbeiten, die die Kohäsion, Klebrigkeit, Stabilität und/oder das Freisetzungsverhalten beeinflussen.

Nachfolgend wird die Erfindung anhand von Ausführungsbeispielen mit Klebern auf Polyacrylat-Basis näher erläutert, diese Beispiele sollen keineswegs beschränkend für den Schutzumfang gedeutet werden.

Klebende Massen auf Acrylatbasis werden durch radikalische Copolymerisation von Acrylsäurederivaten und anderen olefinischen Monomeren in Lösung oder Dispersion hergestellt.

Als klebrigmachende Acrylsäurederivate können bspw. eingesetzt werden:

Methyl-, Ethyl-, n-Propyl-, Isopropyl-, n-Butyl-, Isobutyl-, t-Butyl-, Pentyl-, Hexyl-, Heptyl-, Octyl-, Isooctyl-, 2-Ethylhexyl-, Nonyl-, Decyl-, Isodecyl-, Dodecyl-acrylsäure- und/oder Methacrylsäureester.

Als Monomere mit reaktiven Gruppen für eine spätere Nachvernetzung können bspw. verwendet werden:

Acrylsäure, Methacrylsäure, $\beta$-Carboxyethylacrylat, Crotonsäure, Maleinsäure, Fumarsäure, Itaconsäure, Citraconsäure, Monoester der Maleinsäure mit Methyl-, Ethyl-, n-Propyl-, Isopropyl-, n-Butyl, Isobutyl, t-Butyl-, Hexyl-, Cyclohexyl-, 2-Ethylhexyl- , Octyl-, Isooctyl-, Decyl-Dodecyl-, Isodecylalkohol, Maleinsäureanhydrid, Citraconsäureanhydrid, Itaconsäureanhydrid, Maleinsäure-, Fumarsäure- und Itaconsäuremonoamid, Hydroxymethylacrylat, Hydroxymethylmethacrylat, 2-Hydroxyethylacrylat, 2-Hydroxyethylmethacrylat, 2-Hydroxypropylacrylat, 2-Hydroxypropylmethacrylat, Glycidylacrylat, Glycidylmethacrylat und Allylglycidylether.

Bekannte Vernetzungsmittel sind z.B.: Polyisocyanate, Metallchelate, Metallsäureester und Melamin-Formaldehydharze.

Die Nachvernetzung verleiht dem Kleber seine mechanische Stabilität und Kohäsion.

Manchmal kann es vorteilhaft sein, schon während der Polymerisation für eine gewisse Vernetzung zu sorgen. Man erreicht dies durch den Zusatz von geeigneten Divinylverbindungen. Dies können z.B. sein: Divinylbenzol, Butadien-1.3, Adipinsäure- Divinylester, Adipinsäure-Diallylester und Acrylsäure-Vinylester.

Andere, dem Kleber günstige Eigenschaften verleihende Monomere können z.B. sein:

Vinylacetat, Styrol, Methylvinylether, Ethylvinylether, n-Butyl-vinylether, Vinylpyrrolidon.

Da dieser spezielle Kleber basische Eigenschaften besitzen soll, müssen Monomere mit den entsprechenden basischen Gruppen eingesetzt werden. Dies können z.B. - ohne Anspruch auf Vollständigkeit - sein:

t-Butylaminoethyl-, Dimethylaminoethyl-, Dimethylaminomethyl-, t-Butylaminomethyl, Diethylaminoethyl-, Diethylaminomethyl-, Methylethylaminoethyl-, Methylethylaminomethylacrylat bzw. Methacrylat, verschieden substituierte Aminostyrole.

Geeignete Radikalstarter für die Polymerisation sind z.B.: Azoisobutyronitril, Dibenzoylperoxid, Dilaurylperoxid, t-Butylperpivalat, -peroctat und -peracetat.

Als Lösemittel für die Polymerisation kömmen vor allem Aceton, Methylethylketon, Cyclohexanon, Ethylacetat, Butylacetat, Benzol, Toluol, Xylole, Hexan, Heptan und deren Gemische in Frage.

Die Beispiele beschreiben die Herstellung von wirkstoffhaltigen transdermalen Systemen mit verschiedenem Reservoiraufbau. Als Wirkstoff wurde in allen Fällen Bopindololhydrogenmalonat eingesetzt, jedoch befinden sich unter fast allen Wirkstoffgruppen für die verschiedenen Indikationsgebiete Wirkstoffe, die für die transdermale Verabreichung mit oben beschriebenem Aufbau geeignet sind. Beispielhaft seien genannt: Antirheumatika, Analgetika, blutdruckbeeinflussende Mittel, Antiallergika, Antiphlogistika, $\beta$-Rezeptorenblocker, Calciumantagonisten, Antiasthmatika, Dermatika, durchblutungsfördernde Mittel, Geriatrica, Sedativa, Kardiaka, Koronarmittel, Migränemittel, Muskelrelaxantia, Neuraltherapeutika, Hormone und deren Hemmstoffe, Antihyperkinetika und Zytostatika.

Die hergestellten transdermalen Systeme wurden nach einer validierten Methode unter Verwendung von Haut von Nacktmäusen und Nacktratten als Modell für die menschliche Haut untersucht.

Beispiel 1:

Herstellung einer klebenden Zusammensetzung auf Acrylatbasis mit einpolymerisierten basischen Gruppen

Herstellung einer klebenden Zusammensetzung auf Acrylatbasis mit einpolymerisierten basischen Gruppen

1a) 100 g Dimethylaminoethylmethacrylat, 93.5 g 2-Ethylhexylacrylat, 6 g Acrylsäure und 0.6 g Azoisobutyronitril als Radikalkettenstarter werden in 130 g Ethylacetat gelöst und 3 Stunden am Rückfluß erhitzt. Nach Abkühlen auf Raumtemperatur ist der Kleber gebrauchsfertig. Die Viskosität beträgt 2.0 Pa.sec. bei 22 Grad Celsius (Brookfield Viskosimeter).

1b) 40 g Dimethylaminoethylmethacrylat, 52,7 g 2-Ethylhexylacrylat, 7 g Acrylsäure, 0.3 g Acrylsäurevinylester und 0.3 g Azoisobutyronitril werden in 70 g Ethylacetat gelöst und drei Stunden am Rückfluß erhitzt. Nach Abkühlen auf Raumtemperatur ist der Kleber gebrauchsfertig.

1c) 130 g Dimethylaminoethylmethacrylat, 60g 2-Methyl-Heptylacrylat, 10 g n-Propylester der Acrylsäure, o,3 g Acrylsäurevinylester und 0.3 g AIBN werden in 140 g Ethylacetat gelöst und drei Stunden am Rückfluß erhitzt. Nach Abkühlen auf Raumtemperatur ist der Kleber gebrauchsfertig.

1d) 60 g Dimethylaminoethylmethacrylat, 130g n-Dodecylacrylat, 10 g n-Propylacrylat und 0.3 g AIBN werden in 100 g Ethylacetat gelöst und 3 Std.am Rückfluß erhitzt. Nach Ab-kühlen auf Raumtemperatur ist der Kleber gebrauchsfertig.

1e) 50 g Diethylaminoethylmethacrylat, 140 g i-Amyl-acrylat 10 g Methacrylsäuremethylester, 1 g Methacrylsäurevinylester und 0.3 g AIBN werden in 150 g Methylethylketon gelöst und 3 Stunden am Rückfluß gekocht. Nach Abkühlen auf Raumtemperatur ist der Kleber gebrauchsfertig.

1f) 80 g Diethylaminoethylmethacrylat, 80 g Methacrylsäure-n-butylester 40 g von Methacrylsäureethylester und 1 g Methacrylsäurevinylester und 0.3 g AIBN werden in 100 g Ethylacetat gelöst und 3 Std am Rückfluß gekocht. Nach Abkühlen auf Raumtemperatur ist der Kleber gebrauchsfertig.

1g) 120 g Diethylaminoethylmethacrylat, 60 g Methacrylsäure-n-butylester, 20 g von Methacrylsäureethylester und 1 g Methacrylsäurevinylester und 0.3 g AIBN werden in 100 g Ethylacetat gelöst und 3 Std am Rückfluß gekocht. Nach Abkühlen auf Raumtemperatur ist der Kleber gebrauchsfertig.

Beispiel 2:(Vergleichsbeispiel)

Herstellung eines transdermalen therapeutischen Systems

79,0 g einer 42 Gew.%-igen Lösung eines Klebers auf Acrylatbasis (DUROTAK 280-2416 der Fa. NATIONAL STARCH CHEMICAL B.V., Niederlande), 0.9 g Triethylcitrat, 12,5 g Bopindololhydrogenmalonat und 40,0 g Methylethylketon werden unter Rühren zusammengegeben und nach vollständiger Durchmischung auf eine silikonisierte, aluminisierte 100 μm dicke Polyesterfolie gestrichen. Nach einer Trocknungszeit von 15 min. bei 50 Grad C. wird mit einer aluminisierten 15 um starken Polyesterfolie abgedeckt.

Bei einer Strichdicke von 100 um ergibt sich ein Flächengewicht von 32 g/m$^2$ und ein Wirkstoffgehalt von 8.4 g/m$^2$.

Beispiel 3:

Herstellung von transdermalen therapeutischen Systemen mit einem einpolymerisierte basische Gruppen aufweisenden Kleber

3a) 52,7 g einer 60 Gew./Gew.%-igen Lösung eines Acrylatklebers in Ethylacetat, wie in Beispiel 1a) beschrieben, 1,6 g einer 7,5 Gew.%-igen Lösung von Titanacetylacetonat in Isopropanol, 0.9 g Triethylcitrat und 12,5 g Bopindololhydrogenmalonat in 40 g Methylethylketon wurden, wie in Beispiel 2 beschrieben, verarbeitet. Das fertige transdermale therapeutische System besaß folgende Eigenschaften:
Strichdicke : 100 μm
Flächengewicht: 32 g/m$^2$
Wirkstoffgehalt: 8.9 g/m$^2$
3b) Unter Verwendung des in Beispiel 1b) beschriebenen Acrylatklebers wurde, wie in Beispiel 3a) und 2 erläutert, ein erfindungsgemäßes therapeutisches System mit folgenden Eigenschaften hergestellt:
Strichdicke : 100 μm
Flächengewicht : 27 g/m$^2$
Wirkstoffgehalt : 7,3 g/m$^2$

In vitro-Freisetzungs-Versuche mit nach den Beispielen 2 und 3 hergestellten transdermalen Systemen

Nach den Beispielen 1 bis 3 hergestellte transdermale therapeutische Systeme wurden nach dem von T.J. Franz, J. Invest. Dermatol. 1975 (64), S. 191-195 beschriebenen Verfahren an der Haut von Nacktmäusen und Nacktratten getestet. Die Ergebnisse sind in der nachfolgenden Tabelle zusammengestellt:

| Therap.System (Beispiel) | Bopindololhydrogenmalonat | Penetriert μg/cm$^2$ | | |
|---|---|---|---|---|
| | | 8 h | 24 h | 0-24 h |
| 2 | 0,84 | 10 | 10 | 20 [1] |
| 3a | 0.89 | 30 | 100 | 130 [1] |
| 3a | 0,89 | | | 55 [2] |
| 3b | 0,73 | | | 67 [2] |

[1] Haut von Nacktmäusen
[2] Haut von Nacktratten

**Patentansprüche**

1. Kombination einer basischen wirkstoffdurchlässigen haftklebenden Polymermasse mit basischen Wirkstoffen, wobei die haftklebende Polymermasse ein unter Verwendung von basischen Monomeren hergestelltes Homo- oder Copolymeres, bevorzugt auf Acrylatbasis, ausgeschlossen die Kombination wirkstoffdurchlässiger hydrophiler quellbarer basischer haftklebender Polymere mit Bopindolol, Tizanidin, Clemastin, Ketotifen oder Methysergid, ist.

2. Kombination einer basischen Polymermasse mit basischem Wirkstoff nach Anspruch 1, dadurch gekennzeichnet, daß sie erhältlich ist durch an sich bekannte Polymerisation von:
   a) zwischen etwa 30 bis 70 Gew.%, bevorzugt 40 bis 60 Gew.% eines oder mehrerer Alkylester der Acryl- und/oder Methacrylsäure mit 4 - 12 C-Atomen im Alkylrest;
   b) 5 bis 85 Gew.%, bevorzugt 15 bis 50 Gew.% eines oder mehrerer Aminoester der Acryl- und/oder Methacrylsäure oder anderer in ein Polyacrylat einpolymerisierbarer Olefine mit basischen Gruppen;
   c) 0 bis 50 Gew.%, bevorzugt 5 bis 20 Gew.% eines oder mehrerer Alkylester der Acryl- und/oder Methacrylsäure mit 1 bis 3 C-Atomen im Alkylrest;
   d) 0 bis 15 Gew.%, bevorzugt 0,5 bis 12,5; besonders bevorzugt 1,0 bis 9 und ganz besonders bevorzugt 5 bis 8 Gew.% eines oder mehrerer einpolymerisierbarer Monomere mit für Nachvernetzung geeigneten, reaktionsfähigen Gruppen;
   e) 0 bis 30 Gew.%, bevorzugt 2 bis 15 Gew.% von anderen einpolymerisierbaren Vinylverbindungen; und
   f) 0 bis 5 Gew.%, bevorzugt 0,3 Gew.% von einpolymerisierbaren Divinylverbindungen.

3. Kombination einer basischen Polymermasse mit basischem Wirkstoff nach einem der vorangehenden Ansprüche, dadurch gekennzeichnet, daß der Acrylsäureester mit 1 bis 3 C-Atomen im Alkylrest ausgewählt ist aus der Gruppe bestehend aus Methyl-, Ethyl-, n-Propyl-Isopropyl-Acrylsäureester und/oder Methacrylsäureester; und der Acrylsäure- und/oder Methacrylsäureester mit 4 bis 12 C-Atomen im Alkylrest ausgewählt ist aus der Gruppe bestehend aus n-Butyl-, Isobutyl-, t-Butyl-, Pentyl-, Hexyl-, Heptyl-, Octyl-, Isooctyl-, 2-Ethylhexyl-, Nonyl-, Decyl-, Isodecyl-, Dodecyl-Acrylsäureester und/oder Methacrylsäureestern.

4. Kombination einer basischen Polymermasse mit basischem Wirkstoff nach einem der vorangehenden Ansprüche, dadurch gekennzeichnet, daß das Monomere für spätere Nachvernetzung ausgewählt ist aus der Gruppe bestehend aus: Acrylsäure, Methacrylsäure, β-Carboxyethylacrylat, Crotonsäure, Maleinsäure, Fumarsäure, Itaconsäure, Citraconsäure, Monoester der Maleinsäure mit Methyl-, Ethyl-, n-Propyl-, Isopropyl-, n-Butyl-, i-Butyl-, t-Butyl-, Hexyl-, Cyclohexyl-, 2-Ethylhexyl-, Octyl- Isooctyl-, Decyl-, Dodecyl-, Isodecylalkohol, Maleinsäureanhydrid, Citraconsäureanhydrid, Itaconsäureanhydrid, Maleinsäuremonoamid, Fumarsäuremonoamid und Itaconsäuremonoamid, Hydroxymethylacrylat, Hydroxymethylmethacrylat, 2-Hydroxyethylacrylat, 2-Hydroxyethylmethacrylat, 2-Hydroxypropylacrylat, 2-

6

EP 0 272 562 B1

Hydroxypropylmethacrylat, Glycidylacrylat, Glycidylmethacrylat und Allylglycidylether.

5. Kombination einer basischen Polymermasse mit basischem Wirkstoff nach einem der vorangehenden Ansprüche, dadurch gekennzeichnet, daß der Vernetzer ausgewählt ist aus der Gruppe bestehend aus Polyisocyanaten, Metallchelaten, Metallsäureestern und Melamin-Formaldehydharzen.

6. Kombination einer basischen Polymermasse mit basischem Wirkstoff nach einem der vorangehenden Ansprüche, dadurch gekennzeichnet, daß die Divinylverbindung ausgewählt ist aus der Gruppe bestehend aus Divinylbenzol, Butadien-1.3, Adipinsäure-Divinylester, Adipinsäure-Diallylester und Acrylsäure-Vinylester.

7. Kombination einer basischen Polymermasse nach einem der vorangehenden Ansprüche, dadurch gekennzeichnet, daß die einpolymerisierbare Vinylverbinge(en) ausgewählt ist/sind aus der Gruppe bestehend aus: Vinylacetat, Styrol, Methylvinylether, Ethylvinylether, n-Butylvinylether, Vinylpyrrolidon.

8. Kombination einer basischen Polymermasse mit basischem Wirkstoff nach einem der vorangehenden Ansprüche, dadurch gekennzeichnet, daß die basische Verbindung ausgewählt ist aus der Gruppe bestehend aus t-Butylaminoethyl-, Dimethylaminoethyl-, Dimethylaminomethyl-t-Butylaminomethyl, Diethylaminoethyl-, Diethylaminomethyl-, Methylethylaminoethylacrylat, Methylethylaminomethylacrylat bzw. -methacrylat und verschieden substituierten Aminostyrolen.

9. Verfahren zur Herstellung einer Zusammensetzung nach einem der vorangehenden Ansprüche, gekennzeichnet durch Vermischen von zur Herstellung eines Klebers durch Polymerisation vorgesehenen Monomeren mit einpolymerisierbaren, basischen Monomeren und Copolymerisation des Gemisches in an sich bekannter Weise.

10. Verwendung der Zusammensetzung nach einem der vorangehenden Ansprüche für therapeutische Systeme mit basischen Wirkstoffen, insbesondere solchen in Salzform, bei denen das therapeutisch wirksame Agens eine Lewis-Base ist.

11. Verwendung nach Anspruch 10, dadurch gekennzeichnet, daß der therapeutisch wirksame Teil des Wirkstoffsalzes ein oder mehrere basische Stickstoffatome enthält.

12. Verwendung nach einem der Ansprüche 10 oder 11, dadurch gekennzeichnet, daß der Wirkstoff Bopindolol ist.

**Claims**

1. Combination of a basic adhesive polymeric substance, which is permeable to active materials, with basic active materials, the adhesive polymeric material being a homo- or copolymer which is preferably based on acrylate and is produced by using basic monomers, except the combination of hydrophilic, swellable, basic, adhesive polymers, which are permeable to active materials, with bopindolol, tizanidine, clemastine, ketotifene or methysergide.

2. Combination of a basic polymeric substance with basic active material according to claim 1, characterised in that it is obtainable by means of polymerisation, which is known per se, of:
   a) between about 30 to 70 % by wt., preferably 40 to 60 % by wt., of one or a plurality of alkyl esters of acrylic and/or methacrylic acid having 4 - 12 C atoms in the alkyl residue;
   b) 5 to 85 % by wt., preferably 15 to 50 % by wt., of one or a plurality of amino esters of acrylic and/or methacrylic acid or other olefins, which are polymerisable into a polyacrylate, with basic groups;
   c) 0 to 50 % by wt., preferably 5 to 20 % by wt., of one or a plurality of alkyl esters of acrylic and/or methacrylic acid having 1 to 3 C atoms in the alkyl residue;
   d) 0 to 15 % by wt., preferably 0.5 to 12.5, more especially preferably 1.0 to 9 and most especially preferably 5 to 8 % by wt., of one or a plurality of polymerisable monomers with reactive groups suitable for subsequent cross-linking;
   e) 0 to 30 % by wt., preferably 2 to 15 % by wt., of other polymerisable vinyl compounds; and
   f) 0 to 5 % by wt., preferably 0.3 % by wt., of polymerisable divinyl compounds.

7

3. Combination of a basic polymeric substance with basic active material according to one of the preceding claims, characterised in that the acrylic acid ester having 1 to 3 C atoms in the alkyl residue is selected from the group including methyl-, ethyl-, n-propyl-, isopropyl- acrylic acid ester and/or methacrylic acid ester; and the acrylic acid and/or methacrylic acid ester having 4 to 12 C atoms in the alkyl residue is selected from the group including n-butyl-, isobutyl-, t-butyl-, pentyl-, hexyl-, heptyl-, octyl-, isooctyl-, 2-ethylhexyl-, nonyl-, decyl-, isodecyl-, dodecylacrylic acid ester and/or methacrylic acid esters.

4. Combination of a basic polymeric substance with basic active material according to one of the preceding claims, characterised in that the monomer for subsequent cross-linking is selected from the group including: acrylic acid, methacrylic acid, β-carboxyethyl acrylate, crotonic acid, maleic acid, fumaric acid, itaconic acid, citraconic acid, monoesters of maleic acid with methyl-, ethyl-, n-propyl-, isopropyl-, n-butyl-, i-butyl-, t-butyl-, hexyl-, cyclohexyl-, 2-ethylhexyl-, octyl-, isooctyl-, decyl-, dodecyl-, isodecyl alcohol, maleic acid anhydride, citraconic acid anhydride, itaconic acid anhydride, maleic acid monoamide, fumaric acid monoamide and itaconic acid monoamide, hydroxymethyl acrylate, hydroxymethyl methacrylate, 2-hydroxyethyl acrylate, 2-hydroxyethyl methacrylate, 2-hydroxypropyl acrylate, 2-hydroxypropyl methacrylate, glycidyl acrylate, glycidyl methacrylate and allyl glycidyl ether.

5. Combination of a basic polymeric substance with basic active material according to one of the preceding claims, characterised in that the cross-linking agent is selected from the group including polyisocyanates, metal chelates, metallic acid esters and melamine formaldehyde resins.

6. Combination of a basic polymeric substance with basic active material according to one of the preceding claims, characterised in that the divinyl compound is selected from the group including divinyl benzene, butadiene-1.3, adipic acid divinyl ester, adipic acid diallyl ester and acrylic acid vinyl ester.

7. Combination of a basic polymeric substance according to one of the preceding claims, characterised in that the polymerisable vinyl compound(s) is (are) selected from the group including: vinyl acetate, styrene, methyl vinyl ether, ethyl vinyl ether, n-butyl vinyl ether, vinyl pyrrolidone.

8. Combination of a basic polymeric substance with basic active material according to one of the preceding claims, characterised in that the basic compound is selected from the group including t-butyl aminoethyl-, dimethyl aminoethyl-, dimethyl aminomethyl-t-butyl aminomethyl-, diethyl aminoethyl-, diethyl aminomethyl-, methyl ethyl aminoethyl acrylate, methyl ethyl aminomethyl acrylate or methacrylate and various substituted amino styrenes.

9. Method of producing a composition according to one of the preceding claims, characterised by mixing monomers, which are provided for producing an adhesive by means of polymerisation, with polymerisable, basic monomers and copolymerisation of the mixture in a manner known per se.

10. Use of the composition according to one of the preceding claims for therapeutic systems with basic active materials, more especially those in salt form, wherein the therapeutically active agent is a Lewis base.

11. Use according to claim 10, characterised in that the therapeutically active part of the active material salt contains one or a plurality of basic nitrogen atoms.

12. Use according to one of claims 10 or 11, characterised in that the active material is bopindolol.

**Revendications**

1. Combinaison d'une masse polymère adhésive basique perméable aux principes actifs avec des principes actifs basiques, dans laquelle la masse polymère adhésive est un homo ou un copolymère préparé par utilisation de monomères basiques, de préférence à base d'acrylates, à l'exclusion de la combinaison de polymères adhésifs basiques gonflants hydrophiles perméables aux principes actifs avec du bopindolol, de la tizanidine, de la clémastine, de la cétotifène ou du méthysergide.

**2.** Combinaison d'une masse polymère basique avec un principe actif basique selon la revendication 1, caractérisée en ce qu'elle est préparée par une polymérisation connue en soi des substances suivantes :

a) environ 30 à 70 % en poids, de préférence 40 à 60 % en poids d'un ou plusieurs radicaux alkyle de l'acide acrylique et/ou de l'acide méthacrylique avec 4 à 12 atomes de carbone dans le groupe alkyle,

b) 5 à 85 % en poids, de préférence 15 à 60 % en poids d'un ou plusieurs aminoesters de l'acide acrylique et/ou de l'acide méthacrylique ou d'autres oléfines polymérisables en polyacrylate avec des groupes basiques,

c) 0 à 50 % en poids, de préférence 5 à 20 % en poids d'un ou plusieurs esters alkyliques de l'acide acrylique et/ou de l'acide méthacrylique avec 1 à 3 atomes de carbone dans le radical alkyle,

d) 0 à 15 % en poids, de préférence 0,5 à 12,5 % en poids, particulièrement 1,5 à 9 % en poids et tout particulièrement 5 à 8 % en poids d'un ou plusieurs monomères polymérisables avec des groupes réactifs convenant à une postréticulation,

e) 0 à 30 % en poids, de préférence 2 à 15 % en poids d'autres composés de vinyle polymérisables, et

f) 0 à 5 % en poids, de préférence 0,3 % en poids de composés de divinyle polymérisables.

**3.** Combinaison d'une masse polymère basique avec un principe actif basique selon l'une quelconque des revendications précédentes, caractérisée en ce que l'ester d'acide acrylique ayant 1 à 3 atomes de carbone dans le radical alkyle est choisi dans le groupe constitué des esters d'acide acrylique et/ou des esters d'acide méthacrylique de méthyle, éthyle, n-propyle et isopropyle, et l'ester d'acide acrylique et/ou d'acide méthacrylique ayant 4 à 12 atomes de carbone dans le radical alkyle est choisi dans le groupe constitué des esters d'acides acryliques et/ou des esters d'acides méthacryliques de n-butyle, isobutyle, t-butyle, pentyle, hexyle, heptyle, octyle, isooctyle, 2-éthylhexyle, nonyle, décyle, isodécyle, dodécyle.

**4.** Combinaison d'une masse polymère basique avec un principe actif basique selon l'une quelconque des revendications précédentes, caractérisée en ce que le monomère destiné à une postréticulation est choisi dans le groupe constitué de l'acide acrylique, de l'acide méthacrylique, du $\beta$-carboxyléthylacrylate, de l'acide crotonique, de l'acide maléique, de l'acide fumarique, de l'acide itaconique, de l'acide citrique, du monoester de l'acide maléique avec de l'alcool méthylique, éthylique, n-propylique, isopropylique, i-butylique, t-butylique, hexylique, cyclohexylique, 2-éthylhexylique, octylique, isooctylique, décylique, dodécylique et isododécylique, l'anhydride d'acide maléique, l'anhydride d'acide citraconique, l'anhydride d'acide itaconique, le monoamide d'acide maléique, le monoamide d'acide fumarique et le monoamide d'acide itaconique, l'acrylate d'hydroxyméthyle, le méthacrylate d'hydroxyméthyle, l'acrylate de 2-hydroxyéthyle, le méthacrylate de 2-hydroxyéthyle, l'acrylate de 2-hydroxypropyle, le méthacrylate de 2-hydroxypropyle, l'acrylate de glycidyle, le méthacrylate de glycidyle et l'éther d'allylglycidyle.

**5.** Combinaison d'une masse polymère basique avec un principe actif basique selon l'une quelconque des revendications précédentes, caractérisée en ce que l'agent de réticulation est choisi dans le groupe constitué des polyisocyanates, des agents chélatants de métaux, des esters d'acides métalliques et des résines de mélamine-formaldéhyde.

**6.** Combinaison d'une masse polymère basique avec un principe actif selon l'une quelconque des revendications précédentes, caractérisée en ce que le composé de divinyle est choisi dans le groupe constitué du divinylbenzène, du butadiène-1,3, de l'ester divinylique de l'acide adipique, de l'ester diallylique de l'acide adipique et de l'ester vinylique de l'acide acrylique.

**7.** Combinaison d'une masse polymère basique avec un principe actif basique selon l'une quelconque des revendications précédentes, caractérisée en ce que le ou les composés de vinyle polymérisables sont choisis dans le groupe constitué de l'acétate de vinyle, du styrène, du méthylvinyléther, de l'éthylvinyléther, du n-butylvinyléther, de la vinylpyrrolidone.

**8.** Combinaison d'une masse polymère basique avec un principe actif basique selon l'une quelconque des revendications précédentes, caractérisée en ce que le composé basique est choisi dans le groupe constitué de l'acrylate de t-butylataminoéthyle, de diméthylaminoéthyle, de diméthylaminoéthyle-t-butylaminométhyle, de diéthylaminoéthyle, de diéthylaminométhyle, de méthyléthylaminoéthyle, de l'acrylate ou du méthacrylate de méthyléthylaminométhyle et de divers aminostyrènes substitués.

9. Procédé de préparation d'une composition selon l'une quelconque des revendications précédentes, caractérisée par le mélange de monomères destinés à la préparation d'une colle par polymérisation avec des monomères basiques polymérisables et par la copolymérisation du mélange de manière connue en soi.

10. Utilisation de la composition selon l'une quelconque des revendications précédentes dans des systèmes thérapeutiques avec des principes actifs basiques, en particulier des principes actifs sous la forme de sels dans lesquels le principe actif thérapeutique est une base de Lewis.

11. Utilisation selon la revendication 10, caractérisée en ce que la partie thérapeutique active du sel de principe actif contient un ou plusieurs atomes d'azote basiques.

12. Utilisation selon l'une quelconque des revendications 10 ou 11, caractérisé en ce que le principe actif est le bopindolol.